# EUROPEAN PATENT APPLICATION

(11) **EP 3 315 966 A1**
(43) Date of publication of application: **02.05.2018**
(21) Application number: 16817859.8
(22) Date of filing: 27.06.2016
(51) Int. Cl.: G01N 33/50

(54) **STANDARD THYROID GLAND HORMONE SOLUTION**

(30) Priority: 29.06.2015 JP 2015130037
(71) Applicant: Fujirebio Inc., Tokyo 163-0410 (JP)
(72) Inventor: IBANEZ, Yukina, Tokyo 163-0410 (JP); UMEDA, Kazuyuki, Tokyo 163-0410 (JP); KITAMURA, Yoshiyuki, Tokyo 163-0410 (JP)
(74) Representative: Keirstead, Tanis Evelyne
(86) International application number: PCT/JP2016/068982
(87) International publication number: WO 2017/002751

(57) **Abstract**

Disclosed are a standard solution of a thyroid hormone which is easily adjusted from lot to lot for consistent performance and a method of preparing a standard curve in an immunoassay using the same. The standard solution of a thyroid hormone contains the thyroid hormones and a cyclodextrin or a derivative thereof in a buffer solution. The method of generating a standard curve includes: preparing a plurality of standard solutions of a thyroid hormone containing the thyroid hormone at different known concentrations and a cyclodextrin or a derivative thereof in a buffer solution; and subjecting each standard solution of the thyroid hormone to the immunoassay to prepare a standard curve based on the relationship between the concentration of the thyroid hormone contained in each standard solution of the thyroid hormone and the signal in the immunoassay.

## Description

### TECHNICAL FIELD

The present invention relates to a standard solution for use in an immunoassay for thyroid hormone.

### BACKGROUND ART

Thyroid hormones such as triiodothyronine (T₃) and thyroxine (T₄) in blood are repeatedly produced and then degraded in synchrony with the metabolic cycle. The concentrations of T₃ and T₄ in blood are measured in clinical tests because those concentrations indicate the presence of various diseases. Moreover, T₃ in its free form (FT₃) and T₄ in its free form (FT₄) are also measured because they are the free thyroid hormones that enter into cells and perform their functions, in spite of the fact that T₃ and T₄ in blood are present mostly bound to binding proteins, and a minority of each thyroid hormone is present in its free form. Specifically, the blood FT₃ concentration is used as an indicator for hyperthyroidism (rise in concentration) as well as for Low T₃ syndrome and hypothyroidism (in these cases, drop in concentration). The blood T₃ concentration is used as an indicator for Basedow disease, early subacute thyroiditis, Plummer's disease, and the like (in these cases, rise in concentration) as well as for severe wasting syndrome, pituitary tumor, Sheehan's syndrome, and the like (in these cases, drop in concentration). Also, the blood FT₄ concentration is used as an indicator for hyperthyroidism and Basedow disease (in these cases, rise in concentration) as well as for hypothyroidism (drop in concentration); and the blood T₄ concentration is used as an indicator for Basedow disease, Plummer's disease, hormone unresponsiveness, and the like (in these cases, rise in concentration) as well as for chronic thyroiditis, iodine deficiency, Sheehan's syndrome, and the like (in all of the above cases, drop in concentration).

The concentrations of these thyroid hormones in blood have conventionally been measured by immunoassays. In the immunoassays, the measurement is performed on, in addition to a sample, standard solutions containing each thyroid hormone at known concentrations for preparing a standard curve. To attain more accurate immunoassay, the standard solution is required to have storage stability but it is a problem of the thyroid hormones that they are unstable in an aqueous solution. Currently, a medium having properties similar to those of blood, the proper environment for thyroid hormones, is used as a medium for the standard solution. Specifically, the medium is normal human serum from which thyroid hormones and similar components have been removed by charcoal treatment (Patent Document 1).

### PRIOR ART DOCUMENT

### Patent Document

Patent Document 1: JP 6-94709 A

### SUMMARY OF THE INVENTION

### PROBLEM TO BE SOLVED BY THE INVENTION

In charcoal-treated normal human serum, which has conventionally been used as a medium for a standard solution of a thyroid hormone, the lot-to-lot variation of human serum as a raw material easily leads to variability in the performance of standard solution (stability, reproducibility) and, thus, it is hard to secure the raw material for the stable supply of standard solutions with equal quality.

Thus, an object of the present invention is to provide a standard solution of a thyroid hormone which is easily adjusted from lot to lot for consistent performance.

### MEANS FOR SOLVING THE PROBLEM

The inventors studied intensively and consequently found that thyroid hormones were successfully stored stably for a long period of time without using human serum by allowing the thyroid hormones to coexist with a cyclodextrin or a derivative thereof and thereby completed the present invention.

That is, the present invention provides a standard solution of a thyroid hormone comprising the thyroid hormone and a cyclodextrin or a derivative thereof in a buffer solution. The present invention also provides a reagent for measuring a thyroid hormone, the reagent comprising the above-described standard solution according to the present invention.

The present invention further provides the use of a liquid comprising a thyroid hormone and a cyclodextrin or a derivative thereof in a buffer solution, as a standard solution of the thyroid hormone.

The present invention still further provides a method of preparing a standard curve in an immunoassay, the method comprising: preparing a plurality of standard solutions of a thyroid hormone containing the thyroid hormone at different known concentrations and a cyclodextrin or a derivative thereof in a buffer solution; and subjecting each standard solution of the thyroid hormone to the immunoassay to prepare a standard curve based on the relationship between the concentration of the thyroid hormone contained in each standard solution of the thyroid hormone and the signal in the immunoassay.

### EFFECTS OF THE INVENTION

According to the present invention, a standard solution of a thyroid hormone which is easily adjusted from lot to lot for consistent performance was provided. Because human serum is not used, the problem as seen in the conventional method that the lot-to-lot variation of serum as a raw material easily leads to variability in performance (stability, reproducibility) is prevented in the standard solution of a thyroid hormone according to the present invention.

### DETAILED DESCRIPTION OF THE INVENTION

As described above, the standard solution of a thyroid hormone according to the present invention is a solution comprising a thyroid hormone and a cyclodextrin or a derivative thereof in a buffer solution.

Examples of the thyroid hormone include T₃, T₄, FT₃ and FT₄. Among these, FT₃ and FT₄ are preferred. The concentration of a thyroid hormone in the standard solution is a known predetermined concentration because it is a standard solution. In the case of FT₃, several concentrations are predetermined normally in the range of 50 pg/mL or less, particularly in the range of 30 pg/mL or less. In the case of FT₄, several concentrations are predetermined normally in the range of 20 ng/dL or less, particularly in the range of 10 ng/dL or less. In the case of T₃, several concentrations are predetermined normally in the range of 15 ng/mL or less, particularly in the range of 8 ng/mL or less. In the case of T₄, several concentrations are predetermined normally in the range of 50 µg/dL or less, particularly in the range of 30 µg/dL or less.

As the cyclodextrin, β-cyclodextrin and γ-cyclodextrin are preferred, although the cyclodextrin is not restricted thereto. Moreover, an alkyl or hydroxyalkyl substitution product is preferred as the cyclodextrin derivative and the number of carbon atoms in the alkyl group or hydroxyalkyl group is preferably 1 to 6. Among them, hydroxypropyl-β-cyclodextrin (HPBC), a cyclodextrin substituted with a hydroxypropyl group, is particularly preferred in view of the storage stability of thyroid hormones. The cyclodextrins or the derivatives thereof (hereinafter also referred to simply as "cyclodextrin compound") are commercially available and, therefore, the commercial product may preferably be used. For the cyclodextrin compound, a single type of the compound may be used or a plurality of types of the compounds may be used in combination.

The concentration of the cyclodextrin compound (the total concentration when a plurality of types of the compounds are used) is preferably in the range of 0.1% to 10% by weight, and more preferably in the range of 1.0% to 5.0% by weight. The cyclodextrin compound can exhibit a sufficient effect on stability at a concentration of not less than 0.1 % by weight. On the other hand, the cyclodextrin compound at a concentration of not more than 10% by weight allows immunoassay of FT₃ and FT₄ to be securely performed without any influence on measured values.

The buffer solution used in the present invention is an aqueous buffer solution containing water as a solvent and is a buffer solution having a pH preferably in the range of 6.5 to 10, more preferably in the range of 7.5 to 10, and still more preferably in the range of 8.5 to 9.5, although the pH is not restricted thereto. Examples of the buffer solution having a buffer capacity in such a pH range include, but are not limited to, buffer solutions such as sodium hydrogen carbonate, CABS (4-(cyclohexylamino)-1-butanesulfonic acid), ethanolamine, AMP (2-amino-2-methyl-1-propanol), CAPSO (N-cyclohexyl-2-hydroxy-3-aminopropanesulfonic acid), methylamine and CAPS (N-cyclohexyl-3-aminopropanesulfonic acid), and these buffer solutions are preferably used. Even a buffer solution having a pH buffering capacity outside the pH range of 6.5 to 10 as in the above-described buffer solutions can be used by adjusting the pH to a range of 6.5 to 10 using a pH modifying agent. For example, other various buffer solutions widely used in immunoassay, such as MES (2-morpholinoethanesulfonic acid, monohydrate) buffer solution and phosphate buffer solution, may also be used.

The standard solution of the present invention may comprise a pH modifying agent as necessary. Examples of the pH modifying agent include inorganic bases such as sodium hydroxide and the pH modifying agent can be used in an amount appropriate to adjust the pH to a range of 6.5 to 10.0.

The standard solution of the present invention may further contain sodium chloride. The concentration of sodium chloride may be similar to the concentration of sodium chloride in blood, that is, about 0.85% to 0.9% by weight.

The standard solution of the present invention may further comprise a blocking agent. The blocking agent is routinely used in immunoassay to prevent non-specific adsorption and examples of the blocking agent include, but are not limited to, bovine serum albumin (BSA), skim milk, gelatin, and the like. When a blocking agent is contained, the concentration is not particularly limited and is appropriately set, and is normally about 1.0% to 3.0% by weight, particularly about 1.5% to 2.5% by weight.

The standard solution of the present invention may comprise an appropriate amount of a widely used antimicrobial agent such as sodium azide to prevent putrefaction.

The standard solution of a thyroid hormone according to the present invention can be used in immunoassays in exactly the same manner as a conventional standard solution of a thyroid hormone. Immunoassays of thyroid hormones *per se* are well known and immunoassay kits therefor are commercially available. The standard solution of the present invention may also be used as a standard solution for those commercially available immunoassay kits.

Because immunoassays of thyroid hormones *per se* are well known and immunoassay kits therefor are commercially available, there is no need here to describe them in detail. Briefly, however, in a preferred immunoassay, for example, for the quantification of FT₃, T₂-coupled particles composed of carrier particles coupled with T₂ (diiodothyronine), a sample containing FT₃, and a labeled anti-T₃ monoclonal antibody are allowed to react; the particles are recovered; and then the labeled anti-T₃ monoclonal antibody bound to the particles is measured to successfully quantify FT₃ in the sample (see Example 1 below). The carrier particles are preferably magnetic particles to easily conduct B/F separation, but are not limited to magnetic particles. Moreover, an enzyme widely used as an enzyme label in immunoassays, such as alkaline phosphatase, may be used as the label. A substrate such as chemiluminescence substrate or coloring substrate widely used in immunoassays may be used as a substrate for the enzyme label used for quantifying the enzyme label. For the quantification of FT₄, T₃-coupled particles, a sample containing FT₄, and a labeled anti-T₄ monoclonal antibody are allowed to react, and the labeled anti-T₄ monoclonal antibody bound to the particles is measured to successfully quantify FT₄ in the sample (see Example 2 below). It should be noted that although these methods are competitive assays based on the competition between the thyroid hormone coupled to particles and the thyroid hormone in the sample, the immunoassays are not limited to competitive assays, and other immunoassays such as sandwich assays and agglutination assays may also be used.

When preparing a standard curve by using the standard solution of a thyroid hormone according to the present invention, a plurality of standard solutions of a thyroid hormone comprising the thyroid hormone at different known concentrations and a cyclodextrin or a derivative thereof in a buffer solution are first prepared. The number of the prepared standard solutions of the thyroid hormone is normally not less than two, preferably not less than four. Although there is no particular upper limit, the number is normally not more than 10.

Next, each standard solution is subjected to the immunoassay as described above and signals are measured. For example, in the above-described competitive assay, the enzyme label is reacted with the substrate of the enzyme and the generated signal (for example, chemiluminescence intensity in the case of a chemiluminescence substrate) is measured.

Next, a standard curve can be prepared by plotting the concentration of the thyroid hormone in each solution along the abscissa and the corresponding measured signal along the ordinate.

The present invention will be specifically described below by way of examples. However, the present invention is not limited to the examples below.

### EXAMPLES

### Example 1: Measurement of FT₃

### Example 1-1: Stability test of standard solutions containing various cyclodextrin compounds

FT₃ standard solutions having a composition indicated below were prepared:

| | |
|---|---|
| MES | 10.7 g/L; |
| NaCl | 8.77 g/L; |
| NaN₃ | 1.00 g/L; |
| 4 N NaOH | 9 mL/L; |

one of various cyclodextrin compounds 0.1 or 1.0% by weight (in the case of no added thyroid hormone (first concentration), not included);
BSA 20.0 g/L;
1 N NaOH a proper quantity (for fine adjustment of the pH to 6.8);
FT₃ 0 pg/mL (no addition, first concentration), 1.95 pg/mL ± 20% (second concentration), or 30 pg/mL (sixth concentration) (prepared at the first, second, and sixth concentrations in the range of the first to sixth concentrations of the standard solutions of FT₃ contained in the LUMIPULSE FT3-III (trade name, manufactured by Fujirebio Inc.) which is a commercially available FT₃ immunoassay kit used in the immunoassay).

Each prepared FT₃ standard solution was stored at -80°C or 37°C for one or two weeks and then the FT₃ in the stored standard solution was quantified by immunoassay. The measurement was performed using the LUMIPULSE FT3-III (trade name, manufactured by Fujirebio Inc.) which is a commercially available FT₃ assay kit, and the commercially available LUMIPULSE G1200 (trade name, manufactured by Fujirebio Inc.) as a measuring apparatus. The immunoassay using the LUMIPULSE FT3-III (trade name, manufactured by Fujirebio Inc.) was performed exactly as described in the package insert of the commercial product.

Specifically, the immunoassay was performed as described below. First, 250 µL of the T₂-coupled ferrite particle suspension included in the kit was reacted at 37°C for 20 minutes with 30 µL of each FT₃ standard solution as prepared above and 50 µL of the alkaline phosphatase (ALP)-labeled anti-T₃ monoclonal antibody (sheep) solution included in the kit. Next, the ferrite particles were recovered with a magnet, the reaction liquid was removed, and then the particles were washed. Subsequently, 200 µL of a substrate liquid (AMPPD, (3-(2'-spiroadamantane)-4-methoxy-4-(3"-phosphoryloxy)phenyl-1,2-dioxetane disodium salt)) was applied to the particles and the resulting mixture was allowed to react at 37°C for 5 minutes. Next, the intensity of the emitting light having the emission maximum at a wavelength of 477 nm was measured. The assay principle is based on the competitive reaction of FT₂ and FT₃ against a single monoclonal antibody, and the amount of luminescence indicates a positive correlation with the amount of FT₂ reacted with the antibody and a negative correlation with the amount of FT₃ present in the reaction system. Thus, a larger amount of luminescence indicates a lower FT₃ concentration.

The results obtained after one-week and two-week storage are shown in Table 1-1 below and Table 1-2 below, respectively. Unless otherwise stated in the present specification, each value in the tables is expressed as a relative value taking the amount of luminescence obtained for the standard solution at each concentration stored at -80°C for the same time period as 100.

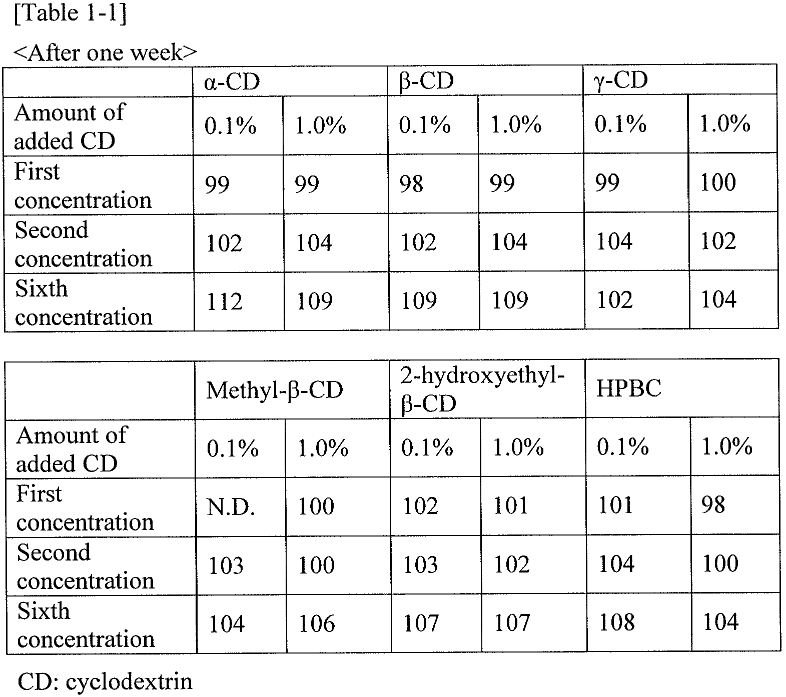

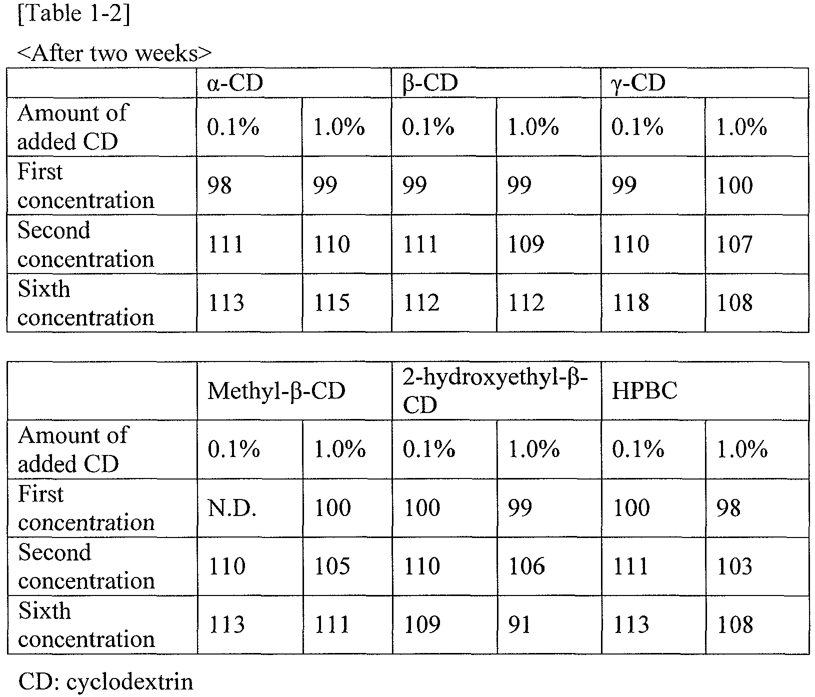

The difference in amount of luminescence from the amount obtained when a standard solution stored at -80°C was used is required to be within ± 20% and is preferably within ± 10% and more preferably within ± 5%. From the results in Table 1, it was indicated that the addition of any cyclodextrin compound caused the FT₃ standard solution to show good stability. Moreover, it was indicated that particularly good stability is obtained with HPBC.

### Example 1-2: Study of optimum pH

FT₃ standard solutions were prepared by the same method as in Example 1-1 except that hydroxypropyl-β-cyclodextrin was used as the cyclodextrin compound, that the concentration was set to 1.0% by weight, and that the pH was set to 6.8, 8.0, or 9.0.

Each prepared FT₃ standard solution was stored at -80°C or 37°C for one week and then the FT₃ in the stored standard solution was quantified by immunoassay. The results are shown in Table 2 below.

**[Table 2]**

| | pH6.8 | | pH8.0 | | pH9.0 | |
|---|---|---|---|---|---|---|
| Amount of added CD | 0.1% | 1.0% | 0.1% | 1.0% | 0.1% | 1.0% |
| First concentration | 101 | 98 | 99 | 98 | 98 | 99 |
| Second concentration | 104 | 100 | 103 | 100 | 104 | 99 |
| Sixth concentration | 108 | 104 | 105 | 102 | 106 | 101 |

From the results in Table 2, it was indicated that a pH in the range of 8.0 to 9.0 was the optimum pH in the FT₃ standard solution.

### Example 1-3 : Study of optimum concentration

FT₃ standard solutions were prepared by the same method as in Example 1-1 except that HPBC or β-cyclodextrin was used as the cyclodextrin compound, that the concentration was set to 0.02% by weight, 0.1% by weight, 0.5% by weight, 1.0% by weight, 5.0% by weight, or 10.0% by weight, and that the pH was set to 8.0.

Each prepared FT₃ standard solution was stored at -80°C or 37°C for one week and then the FT₃ in the stored standard solution was quantified by immunoassay. The results are shown in Table 3 below.

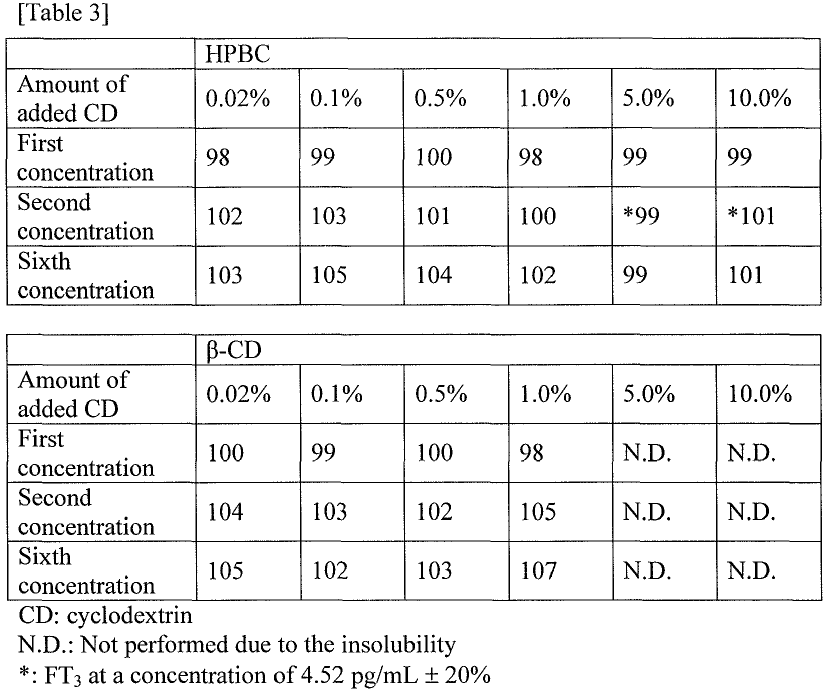

As shown in Table 3, it was indicated that a concentration around 5.0% by weight was the optimum concentration in the case of using HPBC, and a concentration around 0.1% by weight was the optimum concentration in the case of using β-cyclodextrin.

### Example 1-4: Long-term storage stability test

A FT₃ standard solution having a composition indicated below (standard solution A) was prepared. The standard solution A is a standard solution in which conventional charcoal-treated normal human serum is used as a base ingredient.

### <Standard solution A>

To charcoal-treated normal human serum, sodium azide was added to a concentration of 0.1% (w/v) and then T₃ was added to a FT₃ concentration indicated below:
FT₃ 0 pg/mL (no addition, first concentration), 1.95 pg/mL ± 20% (second concentration), or 30 pg/mL (sixth concentration).

On the other hand, a FT₃ standard solution having a composition indicated below (standard solution B1) was prepared. The standard solution B1 is a standard solution according to the present invention.

**<Standard solution B1>**

| | |
|---|---|
| MES | 10.7 g/L; |
| NaCl | 8.77 g/L; |
| NaN₃ | 1.00 g/L; |
| 4 N NaOH | 9 mL/L (used for titration to pH 8.0); |
| HPBC | 1.00% by weight (in the case of no added thyroid hormone (first concentration), not included); |
| BSA | 20.0 g/L; |
| FT₃ | 0 pg/mL (no addition, first concentration), 1.95 pg/mL ± 20% (second concentration), or 30 pg/mL (sixth concentration) |

Each prepared FT₃ standard solution was stored at -80°C, 10°C, or 25°C for one, two, three, four, or five months and then the FT₃ in the stored standard solution was quantified by immunoassay.

The results are shown in Table 4 below.

**[Table 4]**

| | After one month | | After two months | | After three months | | After four months | | After five months | |
|---|---|---|---|---|---|---|---|---|---|---|
| | A | B1 | A | B1 | A | B1 | A | B1 | A | B1 |
| Stored at 10°C First concentration | 100 | 99 | 100 | 99 | 101 | 101 | 100 | 100 | 100 | 100 |
| Second concentration | 101 | 101 | 101 | 101 | 100 | 102 | 100 | 102 | 100 | 102 |
| Sixth concentration | 97 | 102 | 99 | 102 | 102 | 102 | 99 | 102 | 97 | 102 |
| Stored at 25°C First concentration | 101 | 99 | 100 | 99 | 100 | 101 | 100 | 99 | 101 | 98 |
| Second concentration | 100 | 101 | 102 | 101 | 99 | 102 | 101 | 104 | 99 | 105 |
| Sixth concentration | 96 | 102 | 102 | 104 | 99 | 104 | 103 | 107 | 100 | 111 |

As shown in Table 4, the FT₃ standard solutions of the present invention showed excellent long-term storage stability which is proper for practical use, even compared with the conventional serum-based standard solution.

### Example 1-5: Study of buffer solution

A FT₃ standard solution having a composition indicated below (standard solution A) was prepared. The standard solution A is a standard solution in which conventional charcoal-treated normal human serum is used as a base ingredient.

### <Standard solution A>

To charcoal-treated normal human serum, sodium azide was added to a concentration of 0.1% (w/v) and then T₃ was added to a FT₃ concentration indicated below:
FT₃ 0 pg/mL (no addition, first concentration), 1.95 pg/mL ± 20% (second concentration), or 30 pg/mL (sixth concentration).

On the other hand, FT₃ standard solutions each having a composition indicated below (standard solutions B1-B3) were prepared. The standard solutions B1 to B3 are standard solutions according to the present invention.

**<Standard solution B1 (MES: pKa = 6.15)>**

| | |
|---|---|
| MES | 10.7 g/L; |
| NaCl | 8.77 g/L; |
| NaN₃ | 1.00 g/L; |
| 4 N NaOH | (used for titration to pH 8.0); |
| HPBC | 1.00% by weight (in the case of no added thyroid hormone (first concentration), not included); |
| BSA | 20.0 g/L; |
| FT₃ | 0 pg/mL (no addition, first concentration), 1.95 pg/mL ± 20% (second concentration), or 30 pg/mL (sixth concentration) |

**<Standard solution B2 (NaHCO₃: pKa = 10.3)>**

| | |
|---|---|
| NaHCO₃ | 8.40 g/L; |
| NaCl | 8.77 g/L; |
| NaN₃ | 1.00 g/L; |
| 4 N NaOH | (used for titration to pH 9.0); |
| HPBC | 2.00% by weight (in the case of no added thyroid hormone (first concentration), not included); |
| BSA | 20.0 g/L; |
| FT₃ | 0 pg/mL (no addition, first concentration), 1.95 pg/mL ± 20% (second concentration), or 30 pg/mL (sixth concentration) |

**<Standard solution B3 (CAPS: pKa = 10.4)>**

| | |
|---|---|
| CAPS | 22.1 g/L; |
| NaCl | 8.77 g/L; |
| NaN₃ | 1.00 g/L; |
| 4 N NaOH | (used for titration to pH 9.0); |
| HPBC | 2.00% by weight (in the case of no added thyroid hormone (first concentration), not included); |
| BSA | 20.0 g/L; |
| FT₃ | 0 pg/mL (no addition, first concentration), 1.95 pg/mL ± 20% (second concentration), or 30 pg/mL (sixth concentration) |

Each prepared FT₃ standard solution was stored at -80°C, 10°C, or 25°C for six months and then the FT₃ in the stored standard solution was quantified by immunoassay.

The results are shown in Table 5 below.

**[Table 5]**

| | A | B1 | B2 | B3 |
|---|---|---|---|---|
| Stored at 10°C First concentration | 99 | 99 | 99 | 100 |
| Second concentration | 100 | 100 | 100 | 99 |
| Sixth concentration | 99 | 104 | 101 | 100 |
| Stored at 25°C First concentration | 99 | 98 | 98 | 100 |
| Second concentration | 100 | 103 | 98 | 100 |
| Sixth concentration | 100 | 109 | 105 | 108 |

As shown in Table 5, it was suggested that the standard solutions having a pH around 9.0 prepared by using the NaHCO₃ buffer or the CAPS buffer both having a high pKa value had higher stability compared with the standard solution having a pH around 8.0 prepared by using the MES buffer having a low pKa value.

### Example 2: Measurement of FT₄

### Example 2-1: Stability test of a standard solution containing each kind of cyclodextrin compound

A FT₄ standard solution having a composition indicated below was prepared:

| | |
|---|---|
| MES | 10.7 g/L; |
| NaCl | 8.77 g/L; |
| NaN₃ | 1.00 g/L; |
| 4 N NaOH | 9 mL/L; |

one of various cyclodextrin compounds 0.1 or 1.0% by weight (in the case of no added thyroid hormone (first concentration), not included);

| | |
|---|---|
| BSA | 20.0 g/L; |
| 1 N NaOH, | a proper quantity (for fine adjustment of the pH to 6.8); |
| FT₄ | 0 ng/dL (no addition, first concentration), 0.24 ng/dL ± 20% (second concentration), or 10 ng/dL (sixth concentration) (prepared at the first, second, and sixth concentrations in the range of the first to sixth concentrations of the standard solutions of FT₄ contained in the LUMIPULSE FT4-N (trade name, manufactured by Fujirebio Inc.) which is a commercially available FT₄ immunoassay kit used in the immunoassay). |

Each prepared FT₄ standard solution was stored at -80°C or 37°C for one or two weeks and then the FT₄ in the stored standard solution was quantified by immunoassay. The measurement was performed using the LUMIPULSE FT4-N (trade name, manufactured by Fujirebio Inc.) which is a commercially available FT₄ assay kit and the commercially available LUMIPULSE G1200 (trade name, manufactured by Fujirebio Inc.) as a measuring apparatus. The immunoassay using the LUMIPULSE FT4-N (trade name, manufactured by Fujirebio Inc.) was performed exactly as described in the package insert of the commercial product.

Specifically, the immunoassay was performed as described below. First, 250 µL of the T₃-coupled ferrite particles included in the kit were reacted at 37°C for 20 minutes with 10 µL of each FT₄ standard solution as prepared above and 50 µL of the alkaline phosphatase (ALP)-labeled anti-T₄ monoclonal antibody (mouse) included in the kit. Next, the ferrite particles were recovered with a magnet, the reaction liquid was removed, and then the particles were washed. Subsequently, 200 µL of a substrate liquid (AMPPD, (3-(2'-spiroadamantane)-4-methoxy-4-(3"-phosphoryloxy)phenyl-1,2-dioxetane disodium salt)) was applied to the particles, and the resulting mixture was allowed to react at 37°C for 5 minutes. Next, the intensity of the emitting light having the emission maximum at a wavelength of 477 nm was measured. The assay principle is based on the competitive reaction of FT₃ and FT₄ against a single monoclonal antibody, and the amount of luminescence indicates a positive correlation with the amount of FT₃ reacted with the antibody and a negative correlation with the amount of FT₄ present in the reaction system. Thus, a larger amount of luminescence indicates a lower FT₄ concentration.

The results obtained after one-week and two-week storage are shown in Table 6-1 below and Table 6-2 below, respectively. Additionally, each value in the tables is expressed as a relative value taking the amount of luminescence obtained for the standard solution at each concentration stored at -80°C for the same time period as 100.

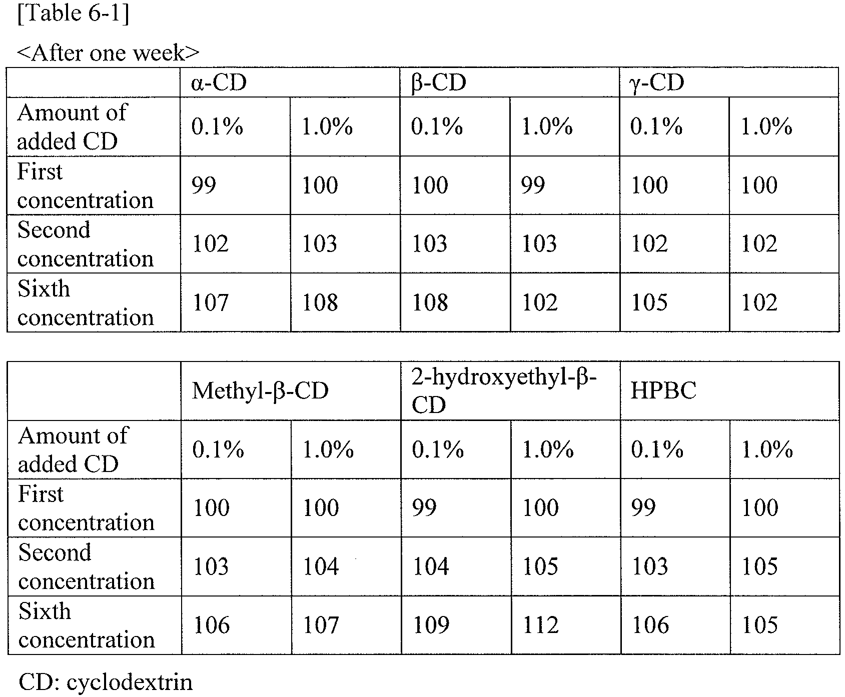

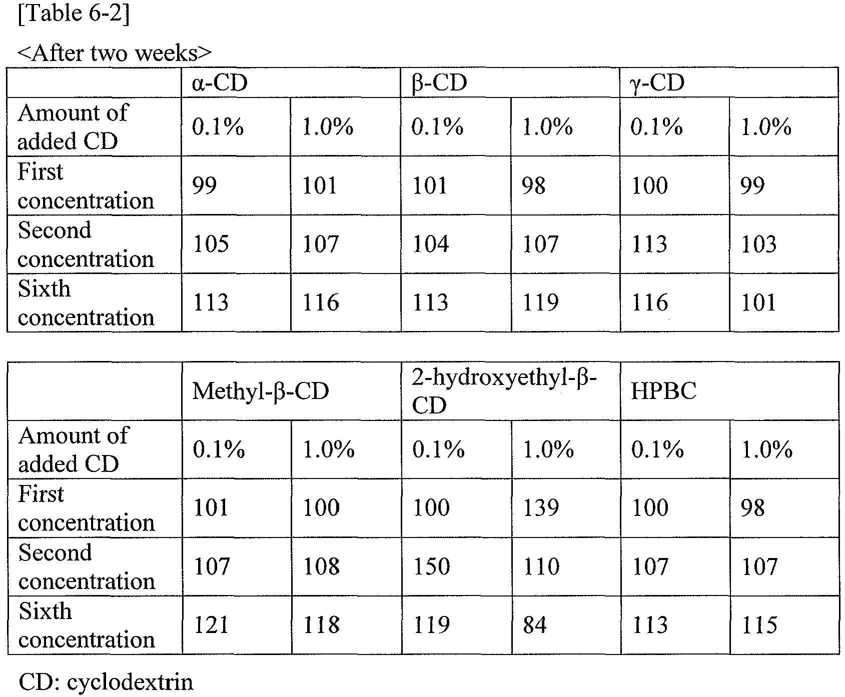

The difference in amount of luminescence from the amount obtained when a standard solution stored at -80°C was used is required to be within ± 20% and is preferably within ± 10% and more preferably within ± 5%. From the results in Table 1, it was indicated that the addition of any cyclodextrin compound caused the FT₄ standard solution to show good stability by adjusting the concentration of the compound. Moreover, it was indicated that particularly good stability is obtained with γ-cyclodextrin or hydroxypropyl-β-cyclodextrin.

### Example 2-2: Study of optimum pH

FT₄ standard solutions were prepared by the same method as in Example 2-1 except that hydroxypropyl-β-cyclodextrin was used as the cyclodextrin compound, that the concentration was set to 1.0% by weight, and that the pH was set to 6.8, 8.0, or 9.0.

Each prepared FT₄ standard solution was stored at -80°C or 37°C for one week and then the FT₄ in the stored standard solution was quantified by immunoassay. The results are shown in Table 7 below.

**[Table 7]**

| | pH6.8 | | pH8.0 | | pH9.0 | |
|---|---|---|---|---|---|---|
| Amount of added CD | 0.1% | 1.0% | 0.1% | 1.0% | 0.1% | 1.0% |
| First concentration | 99 | 100 | 100 | 99 | 100 | 97 |
| Second concentration | 103 | 105 | 101 | 100 | 101 | 95 |
| Sixth concentration | 106 | 105 | 97 | 103 | 98 | 100 |

From the results in Table 7, it was indicated that a pH in the range of 8.0 to 9.0 was the optimum pH in the FT₄ standard solution.

### Example 2-3: Study of optimum concentration

FT₄ standard solutions were prepared by the same method as in Example 2-1 except that HPBC or β-cyclodextrin was used as the cyclodextrin compound, that the concentration was set to 0.02% by weight, 0.1% by weight, 0.5% by weight, 1.0% by weight, 5.0% by weight, or 10.0% by weight, and that the pH was set to 8.0.

Each prepared FT₄ standard solution was stored at -80°C or 37°C for one week and then the FT₃ in the stored standard solution was quantified by immunoassay. The results are shown in Table 8 below.

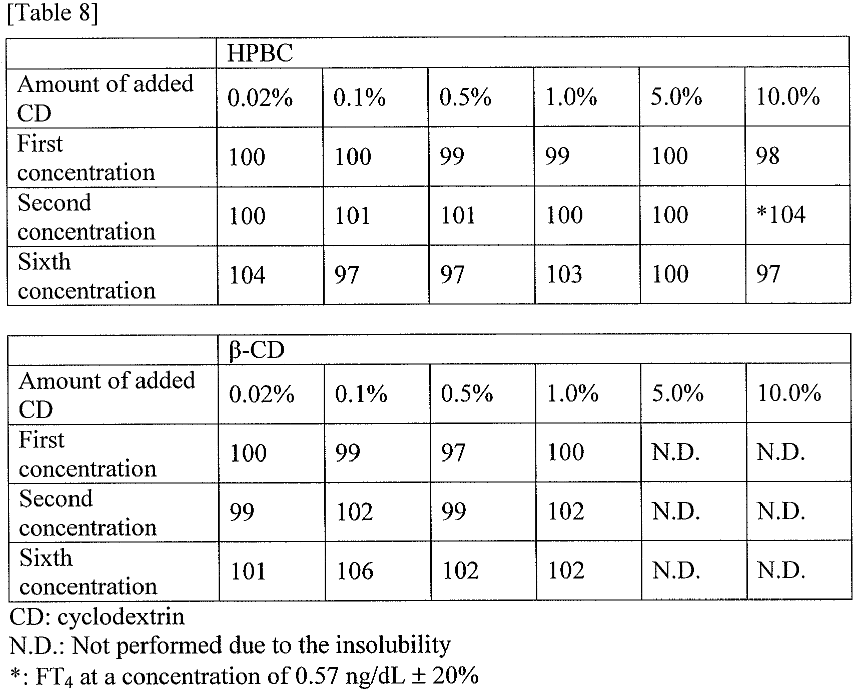

As shown in Table 8, it was indicated that a concentration around 5.0% by weight was the optimum concentration in the case of using HPBC, and a concentration around 0.5% by weight was the optimum concentration in the case of using β-cyclodextrin.

### Example 2-4: Long-term storage stability test

A FT₄ standard solution having a composition indicated below (standard solution C) was prepared. The standard solution C is a standard solution in which conventional charcoal-treated normal human serum is used as a base ingredient.

### <Standard solution C>

To charcoal-treated normal human serum, sodium azide (NaN₃) was added to a concentration of 0.1% (w/v) and then T₄ was added to a FT₄ concentration indicated below:
FT₄ 0 ng/dL (no addition, first concentration), 0.24 ng/dL ± 20% (second concentration), or 10 ng/dL (sixth concentration).

A FT₄ standard solution having a composition indicated below (standard solution D1) was prepared. The standard solution D1 is a standard solution according to the present invention.

**<Standard solution D1>**

| | |
|---|---|
| MES | 10.7 g/L; |
| NaCl | 8.77 g/L; |
| NaN₃ | 1.00 g/L; |
| 4 N NaOH | 9 mL/L; |
| HPBC | 1.00% by weight (in the case of no added thyroid hormone (first concentration), not included); |
| BSA | 20.0 g/L; |
| 1 N NaOH | a proper quantity (for fine adjustment of the pH to 8.0); |
| FT₄ | 0 ng/dL (no addition, first concentration), 0.24 ng/dL ± 20% (second concentration), or 10 ng/dL (sixth concentration) (prepared at the first, second, and sixth concentrations in the range of the first to sixth concentrations of the standard solutions of FT₄ contained in the LUMIPULSE FT4-N (trade name, manufactured by Fujirebio Inc.) which is a commercially available FT₄ immunoassay kit used in the immunoassay) |

Each prepared FT₄ standard solution was stored at -80°C, 10°C, or 25°C for one, two, three, four, or five months and then the FT₄ in the stored standard solution was quantified by immunoassay.

The results are shown in Table 9 below.

**[Table 9]**

| | After one month | | After two months | | After three months | | After four months | | After five months | |
|---|---|---|---|---|---|---|---|---|---|---|
| | C | D | C | D | C | D | C | D | C | D |
| Stored at 10°C First concentration | 99 | 102 | 100 | 100 | 100 | 99 | 99 | 100 | 101 | 98 |
| Second concentration | 99 | 101 | 100 | 101 | 100 | 102 | 101 | 103 | 101 | 99 |
| Sixth concentration | 101 | 102 | 102 | 100 | 101 | 104 | 100 | 101 | 105 | 101 |
| Stored at 25°C First concentration | 100 | 100 | 101 | 101 | 99 | 99 | 99 | 101 | 99 | 99 |
| Second concentration | 100 | 99 | 99 | 101 | 98 | 103 | 99 | 103 | 98 | 100 |
| Sixth concentration | 100 | 102 | 98 | 100 | 99 | 101 | 100 | 102 | 96 | 99 |

As shown in Table 9, the FT₄ standard solutions of the present invention showed excellent storage stability which is sufficient for practical use, even compared with the conventional serum-based standard solution.

### (Example 2-5): Study of composition of buffer solution

A FT₄ standard solution having a composition indicated below (standard solution C) was prepared. The standard solution C is a standard solution in which conventional charcoal-treated normal human serum is used as a base ingredient.

### <Standard solution C>

To charcoal-treated normal human serum, sodium azide (NaN₃) was added to a concentration of 0.1% (w/v) and then T₄ was added to a FT4 concentration indicated below:
FT₄ 0 ng/dL (no addition, first concentration), 0.24 ng/dL ± 20% (second concentration), or 10 ng/dL (sixth concentration)

On the other hand, FT₄ standard solutions each having a composition indicated below (standard solutions D1-D3) were prepared. The standard solutions D1 to D3 are standard solutions according to the present invention.

**<Standard solution D1 (MES: pKa = 6.15)>**

| | |
|---|---|
| MES | 10.7 g/L; |
| NaCl | 8.77 g/L; |
| NaN₃ | 1.00 g/L; |
| 4 N NaOH | (used for titration to pH 8.0); |
| HPBC | 1.00% by weight (in the case of no added thyroid hormone (first concentration), not included); |
| BSA | 20.0 g/L; |
| FT₄ | 0 ng/dL (no addition, first concentration), 0.24 ng/dL ± 20% (second concentration), or 10 ng/dL (sixth concentration) |

**<Standard solution D2 (NaHCO₃: pKa = 10.3)>**

| | |
|---|---|
| NaHPO₄ | 8.40 g/L; |
| NaCl | 8.77 g/L; |
| NaN₃ | 1.00 g/L; |
| 4 N NaOH | (used for titration to pH 9.0); |
| HPBC | 2.00% by weight (in the case of no added thyroid hormone (first concentration), not included); |
| BSA | 20.0 g/L; |
| FT₄ | 0 ng/dL (no addition, first concentration), 0.24 ng/dL ± 20% (second concentration), or 10 ng/dL (sixth concentration) |

**<Standard solution D3 (CAPS)>**

| | |
|---|---|
| CAPS | 22.1 g/L; |
| NaCl | 8.77 g/L; |
| NaN₃ | 1.00 g/L; |
| 4 N NaOH | (used for titration to pH 9.0); |
| HPBC | 2.00% by weight (in the case of no added thyroid hormone (first concentration), not included); |
| BSA | 20.0 g/L; |
| FT₄ | 0 ng/dL (no addition, first concentration), 0.24 ng/dL ± 20% (second concentration), or 10 ng/dL (sixth concentration) |

Each prepared FT₄ standard solution was stored at -80°C, 10°C, or 25°C for six months and then the FT₄ in the stored standard solution was quantified by immunoassay.

The results are shown in Table 10 below.

**[Table 10]**

| | C | D1 | D2 | D3 |
|---|---|---|---|---|
| Stored at 10°C First concentration | 100 | 100 | 99 | 100 |
| Second concentration | 100 | 102 | 101 | 101 |
| Sixth concentration | 98 | 102 | 100 | 100 |
| Stored at 25°C First concentration | 99 | 100 | 100 | 100 |
| Second concentration | 99 | 104 | 101 | 101 |
| Sixth concentration | 100 | 107 | 96 | 99 |

As shown in Table 10, it was suggested that the standard solutions having a pH around 9.0 prepared by using the NaHCO₃ buffer or the CAPS buffer both having a high pKa value had higher stability compared with the standard solution having a pH around 8.0 prepared by using the MES buffer having a low pKa value.

### INDUSTRIAL APPLICABILITY

The present invention provides a standard solution that is important for the diagnosis of various thyroid hormone-related diseases and useful for improving the reliability of the measurement of thyroid hormones in blood samples.

## Claims

1. A standard solution of a thyroid hormone, the standard solution comprising the thyroid hormone and a cyclodextrin or a derivative thereof in a buffer solution.

2. The standard solution according to claim 1, wherein the thyroid hormone is triiodothyronine, free triiodothyronine, thyroxine, or free thyroxine.

3. The standard solution according to claim 1 or 2, wherein the cyclodextrin or the derivative thereof is β-cyclodextrin, γ-cyclodextrin, or an alkyl or hydroxyalkyl substitution product thereof.

4. The standard solution according to claim 3, wherein the cyclodextrin or the derivative thereof is hydroxypropyl-β-cyclodextrin.

5. The standard solution according to any one of claims 1 to 4, wherein the concentration of the cyclodextrin or the derivative thereof is 0.1% to 10% by weight.

6. The standard solution according to any one of claims 1 to 5, wherein the standard solution has a pH of 6.5 to 10.

7. A reagent for measuring a thyroid hormone, the reagent comprising the standard solution according to any one of claims 1 to 6.

8. Use of a liquid comprising a thyroid hormone and a cyclodextrin or a derivative thereof in a buffer solution, as a standard solution of the thyroid hormone.

9. A method of generating a standard curve in an immunoassay, the method comprising: preparing a plurality of standard solutions of a thyroid hormone containing the thyroid hormone at different known concentrations and a cyclodextrin or a derivative thereof in a buffer solution; and subjecting each standard solution of the thyroid hormone to the immunoassay to prepare a standard curve based on the relationship between the concentration of the thyroid hormone contained in each standard solution of the thyroid hormone and the signal in the immunoassay.
